# EUROPEAN PATENT APPLICATION

(11) **EP 4 066 810 A1**
(43) Date of publication of application: **05.10.2022**
(21) Application number: 21204143.8
(22) Date of filing: 22.10.2021
(51) Int. Cl.: A61H 39/04

(54) **METHOD FOR OPERATING HEALTH-CARE DEVICE**

(30) Priority: 31.03.2021 TW 110111869
(71) Applicant: Tao Mining Co., Ltd., Taipei 104 (TW)
(72) Inventor: TSAI, Ching-Fu, Taipei (TW)
(74) Representative: Isarpatent

(57) **Abstract**

A method for operating a health-care device (1) is disclosed. The proposed method includes steps of coaxially aligning a central opening (1705) of a bottom connector (1704) and a specific part or an acupoint of a user's body, causing a device positioning piece (1702, 1940) positioned on the user's body, causing a work body (10, 390, 1724, 1910) mounted on the bottom connector (1704), and operating a work piece (20, 380, 1718, 1730, 1800, 1810, 1820, 1920) to form a working relation between a working end (24, 384, 1770, 1930) thereof and the acupoint or the specific part, and a positional relation therebetween.

## Description

This application claims the benefits of Taiwan Patent Application Number 110111869 filed on March 31, 2021, at the Taiwan Intellectual Property Office, the disclosures of which are incorporated herein in their entirety by reference.

The present disclosure relates to a health-care device, in particular to a method for operating a health-care device.

The present disclosure is a method for operating a health-care device, which is disclosed in an application filed on the same date as the present application and claiming the priorities of PCT/CN2020/088533 filed on April/30/2020 and PCT/CN2020/107950 filed on August/7/2020.

As described in the parallel application of the health-care device of the same filing date for operation by the present application: "Those who lose their health will then know that a life of no disease and no pain is a great blessing". Human nature is usually greedy for life and fear of death, and if possible, longevity is one of the big quests of ordinary people. Everyone seems to agree that prevention is better than cure, but when we face the life journey of birth, aging, sickness and death, not everyone has a chance or a perseverance to pay attention to the prevention all the time. The present disclosure will imitate the most famous doctors in Chinese history, Hua Tuo and Bian Que, and look forward to bringing health and happiness to mankind.

As above-mentioned, the health-care device for operation by the present disclosure and its theories and minute particulars can be seen in the parallel application filed on the same date and its parent applications, and thus the present disclosure only retains the relevant parts of the content as the basis to facilitate the clear description of the operation method and/or mode of implementation of the health-care device.

The health-care device using the method of the present invention is to engage in a health-care work towards a specific body part (which usually is an acupoint or a part feeling unwell). Here, the health-care work is a health care activity made on a specific acupoint or a specific body part to obtain for the subject a health effect. For example, via an electrical stimulation (as described in Paragraphs [0001] and [0019] in the specification of the first basic priority of the parallel application), a light stimulation (which, as described in Paragraph [0002] of the specification of the first basic priority of the parallel health-care device application filed on the same date, includes a laser stimulation (as described in Paragraph [0003] of the specification of the first basic priority)), a thermal stimulation (as described in Paragraph [0004] of the specification of the first basic priority), a non-invasive or a contactless acupoint stimulation mode (as described in Paragraphs [0005] and [0017] of the specification of the first basic priority), a microwave stimulation (as described in Paragraph [0006] and Paragraphs [0021] and [0029] of the specification of the first basic priority), a magnetic stimulation (as described in Paragraphs [0007]-[0038] of the specification of the first basic priority), and/or a traditional acupuncture (as described in Paragraphs [0008] and [0044] of the specification of the first basic priority). One of objectives of the present invention is to provide a method for operating a health-care device to obtain a safety of using the health-care device.

The other objective of the present invention is to provide a method for operating a health-care device to obtain a personalized use of the health-care device.

One more objective of the present invention is to provide a method for operating a health-care device in order to use the mobile device currently everyone has one to assist the user in correctly using the health-care device.

A further objective of the present invention is to provide a method for efficiently operating a health-care device.

The realization of the above-mentioned one or more objectives, or more other objectives can be realized via the embodiments as follows, but each of the embodiments only belongs to an example of the conception of invention.
Fig. 1 is an embodiment of a work piece of a health-care device using an operating method of the present invention.
Fig. 2 is a first embodiment of a health-care device using an operating method of the present invention.
Fig. 3 is a second embodiment of a health-care device using an operating method of the present invention.
Fig. 4 is a third embodiment of a health-care device using an operating method of the present invention.
Fig. 5A is a first embodiment of a work piece of a fourth embodiment of a health-care device using an operating method of the present invention.
Fig. 5B is a second embodiment of a work piece of a fourth embodiment of a health-care device using an operating method of the present invention.
Fig. 5C is a third embodiment of a work piece of a fourth embodiment of a health-care device using an operating method of the present invention.
Fig. 6 is a fifth embodiment of a health-care device using an operating method of the present invention.
Fig. 7 is a first embodiment of a flow chart of steps of an operating method of the present invention.
Fig. 8 is a second embodiment of a flow chart of steps of an operating method of the present invention.
Fig. 9 is an embodiment of a mobile phone screen showing human body parts which may feel unwell or have diseases and/or symptoms for selection.
Fig. 10 is a picture and descriptions of of Quze acupoint of the Pericardium Meridian of Hand-Jueyin.
Fig. 11 is a round sticker helping the user to identify a location of an acupoint.

### DETAILED DESCRIPTION OF THE PREFERRED EMBODIMENT

The working principles of the relevant health-care device and the relevant embodiments regarding how to operate the health-care device are successively disclosed cooperating with the drawings as follows. Regarding the health-care device, more of its embodiments can be seen in the parallel health-care device application filed on the same date. The present disclosure only discloses few embodiments possibly enough to reveal the present operating method, and more efforts are put on full and clear descriptions of the specific embodiments.

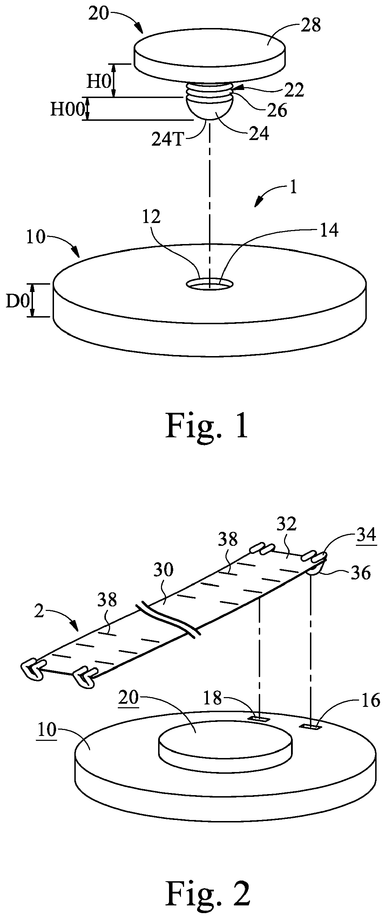

Please refer to Fig. 1, according to the first embodiment of the acupoint working device or a health-care device 1 including a work body 10 and a work piece 20, work body 10 has a central screw hole 12, an internal thread 14 and a thickness D0. Work piece 20 has a working rod 22 and an operating part 28. Working rod 22 has a working end 24 and a threaded part 26. Threaded part 26 has a height H0 (a length extended downward from a bottom surface of the operating part 28) being a little bit larger than thickness D0 of work body 10 to facilitate the connection with internal thread 14, and to decide afterward an extent that working end 24 protrudes over work body 10. Operating part 28 is for manual operation to decide the non-invasive depth or strength that working end 24 presses or works on the acupoint.

The feeling of the light or heavy pressures borne by the acupoint might vary significantly with (1) age, (2) physique and (3) a coarseness or a tenderness of the skin and/or muscle texture. As we know, the pressure is obtained from (4) force divided by (5) area, that is, in addition to the force magnitude, the bearing area is also playing a determining factor of equal importance. The pressure values of the acupoint in the following table have included the full scope for the five factors above, and two embodiments of the bearing area show the corresponding force values.

| Pressing type | Pressure on acupoint | Force borne on an area of 0.03cm² | Force borne on an area of 0.07cm² |
|---|---|---|---|
| Light | 3-12 kgf/cm² | 0.09-0.36kgf/0.882-3.528N | 0.21-0.84kgf/2.058-8.232N |
| Heavy | 8-18 kgf/cm² | 0.24-0.54kgf/2.352-5.292N | 0.56-1.26kgf/5.488-12.348N |

| | | | |
|---|---|---|---|
| *1kgf=9.8N | | | |

General speaking, when we press the acupoint with our finger, the action area is about 0.3 cm² (the real action area of the finger is 1cm^{∗}0.3cm), so the force pressing the acupoint, light or heavy, is about 0.9-5.4kgf. It is this force that causes the person applying the finger pressure to feel fatigue. If we use the tip of working end 24 in Fig. 1 (no matter whether it is 0.03cm² or 0.07cm²) to do this, the required force will only be 0.09-1.26kgf (even if the action area is enlarged to 0.2cm², the required force will only be 0.6-3.6kgf). Not only it is obviously much easier, but also because it does not borrow the manpower or something else to do it, so one will not feel fatigue, and thus it can serve the user at any time. The tip of working end 24 is better bluntly rounded. If its full action area is less than 0.02cm², it is easy to cause pain, and to those with weaker muscles, it may be hurtful, so it should not be less than 0.02cm². On the other hand, if its full action area is larger than 0.8cm², it is unlikely to cause the pain feeling, but a precise interaction with the acupoint may become difficult. Thus, a better range of the full action area may be between 0.05cm² and 0.6cm².

In other words, the present invention uses working end 24 to serve as the hand of the Chinese physician to work on the acupoint. What are differences between treatment effects of the acupuncture and the finger pressure? The experienced Chinese physicians (such as Pan Longsen) will answer: if we can precisely get the acupoint, the effect of finger press can achieve up to 80% of that of acupuncture. Here, we can further analyze as follows: although the effect of finger pressure is only 80% of that of acupuncture, the acupuncture time is limited and quite expensive. If the user owns the acupoint working device 1, by lengthening the pressing time, it can turn defeat into victory through the cumulative effect (instant effect^{∗}application time).

Upon using health-care device 1, as shown in Fig. 2, work body 10 further includes two grooves 16, each having an inclined hook portion 18. A flexible health-care body 30, being a cloth tape or strip 30 here has two ends 32, each provided with two hooks 34, each of which has an inclined hook end 36 to hook with hook portion 18. Cloth tape 30 is configured with a plurality of buckling line segments 38 near each end 32 such that cloth tape 30 is used to wrap health-care device 1 around the user. According to a specific body size and the remaining length of the last loop, the users selects the most appropriate line segment pair 38 to fix acupoint working device 1 so that acupoint working device 1 and a specific acupoint may maintain therebetween a positioning relationship. Thus, acupoint working device 1 and cloth tape 30 commonly form a health-care device 2, which is comparable to various health-care devices in the parent application of the parallel application. Certainly, for the sake of beauty, cloth tape 30 can be silk, or any other delightful quality or design.

In use, flexible health-care body 30 is to fix health-care device 1 to the body part such that working end 24 and the acupoint maintain a positioning relationship with each other, wherein the body part has a skin surface. A first end 32 of flexible health-care body 30 can be connected to the work device 10, or simply put on the skin surface or the health-care device 1. This is because flexible health-care body 30 belongs to a cloth quality, so that after self-entanglement for several times, first end 32 is self-restrained because of accumulative frictions with work body 10 (and/or work piece 20 or the surface of skin). For reliable purposes, flexible health-care body 30 sew or configure the plurality of buckling line segment pairs 38 on the surface near another end 32 for hooking hooks 34. Thus, limited by the specific relationship, a tip 24T of working end 24 uses a working depth downward of the skin surface to engage in a pressing work or a health-care work.

According to plumpness and different body part, finger or working end 24 depresses the skin surface in a depth about 0.1-3cm. Preferably, or for a normal body type, it is between 0.2-2cm. For all body parts, a more preferably or common working depth is usually in a range of 0.3-1.5cm. Thus, if H00 of working end 24 is set at 0.2cm, we can obtain H0 (a height of threaded part 26) is a little bit larger than D0 (a thickness of work body 10), meaning it (H0) should not exceed 1.8cm. Because the acupoints of the chest and back are not far away from the organs, for these acupoints, the drop that tip 24T (see Fig. 1) is below the surface of skin should not exceed 0.3cm. Certainly, H0 for these acupoints should better be equal to D0 to ensure the safety. To explain in detail, although the working depth of work piece 20 can be completely defined by the range of H00 of working end 24, in order to fit or attach operating part 28 with or to the work body 10 to neatly operate health-care device 1 on the surface of the skin, threaded part 26 is better completely locked into internal thread 14. Thus, the working depth of health-care device 1 is better determined by threaded part 26 and/or working end 24 protruding beyond the lower surface of work body 10, and the range of H00 is at least 0.1-0.3cm to prevent threaded part 26 from hurtfully contacting the skin. Certainly, at the body part suitable for a deeper working depth, because the present invention is non-invasive, if H00 is set between 0.3-0.5cm, then H0 of threaded part 26 being larger than the thickness of work body 10 can be obtained in the range of 1.5-1.7cm (assuming the total work depth is in the range of 0.2-2cm). When viewed from another angle, if the working depth is fully executed by working end 24, under the total working depth, the length of working end 24 can reach 2cm. On the contrary, if the working depth is mainly performed by threaded part 26, considering the circumstances that the height of working end 24 is preferably 0.3cm, the height of threaded part 26 larger than the thickness of work body 10 should be less than 1.7cm. But, considering the adjustment demands of the work depth at different acupoints, the better or more common range of threaded part 26 should be between 0.3-1.5cm.

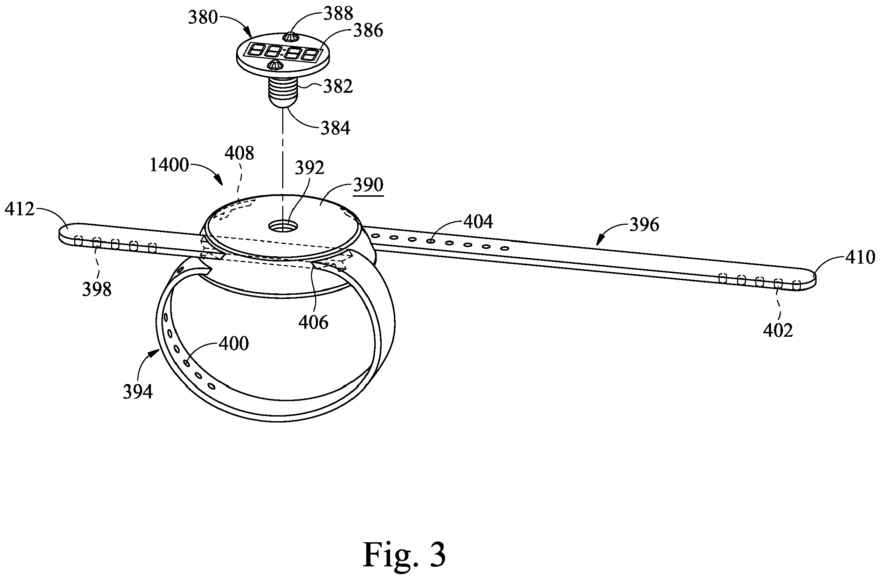

Please refer to Fig. 3, the second embodiment of the health-care device 1400 includes a work body 390 and a work piece 380, wherein work body 390 includes a central screw hole 392 and two symmetrical side wings 394/396. Right wing 396 has a plurality of bottom buckles 402 and a plurality of mating grooves 404, and left wing 394 has a plurality of bottom buckles 398 and a plurality of grooves 400. Work body 390 has two symmetrical transverse through grooves 406/408 such that two free ends of two wings 394/396 can respectively pass therethrough to be self-buckled and positioned. Two wings 394/396 can thus adjust a perimeter formed thereby and surrounding the body part via mutually buckled bottom buckles/grooves 398/400 (402/404). Work body 390 can be all transparent to facilitate the user to position the acupoints. Work piece 380 includes an threaded part 382, a working end 384, a timer 386 and a jewel 388. Upon using, pre-assembling wings 394/396 on work body 390, as shown by left wing 394, and pre-aligning central screw hole 392 with an acupoints of a specific body part, because work body 390 can further be partly or fully transparent, it is easy to know the alignment status, and then work piece 380 can be screwed into screw hole 392 for eventually working or pressing on the accurate acupoint. Certainly, these wings 394/396 can be one-piece formed with work body 390. This wing design may certainly be applied to the preceding embodiments.

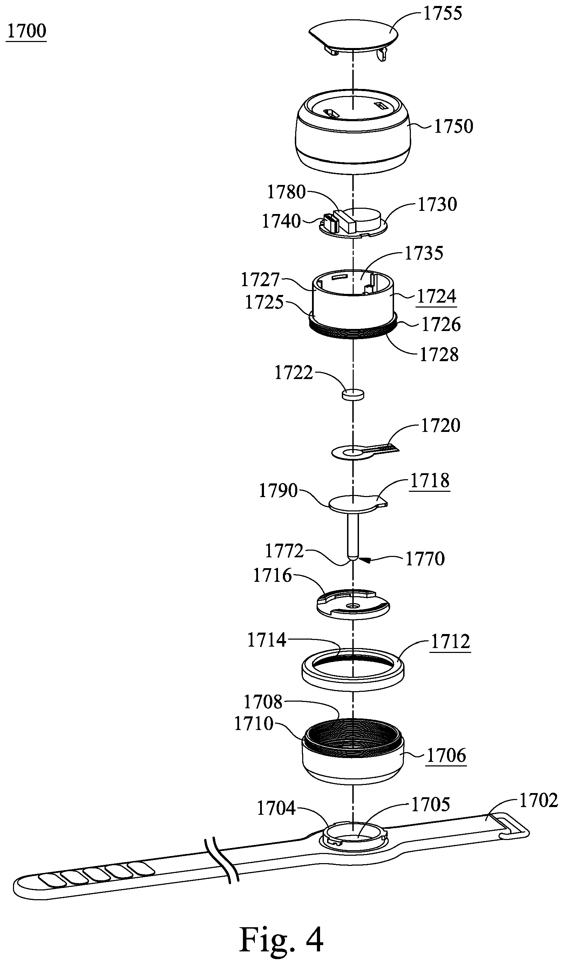

Please refer to Fig. 4, a further embodiment of the health-care device 1700 includes device positioning piece being a flexible piece or a strap 1702, a bottom connector (e.g., a bottom fastener) 1704 connected to strap 1702 and having a central opening 1705, a lower shell 1706 engaging with bottom fastener 1704 and having an internal thread 1708 and an external thread 1710, a lower shell fastener 1712 having an internal thread 1714, an upper shell fastener 1716, a work piece 1718 having an operating end 1790 and an opposite working end 1770, a working extent sensor 1720, a positioning piece 1722, an upper shell 1724 having an upper part 1727, a lower part 1725, an external thread 1726, a bottom surface 1728 and a containing space 1735, a circuit board 1730 electrically connected to working extent sensor 1720 to engage in sensing, a lithium battery 1780 configured on circuit board 1730, a USB charging socket 1740 configured on circuit board 1730 to charge circuit board 1730, an upper cover 1750 and a top cover 1755.

Upon assembling, circuit board 1730, lithium battery 1780, and USB charging socket 1740 are positioned or put into containing space 1735 first, and then work piece 1730, lithium battery 1780, working extent sensor 1720 and positioning piece 1722 are superimposed sequentially to be fastened to bottom surface 1728 via upper shell fastener 1716 to form a fastened component. Then, this fastened component is put into lower shell 1706 for protruding downward working end 1770 (having a tip 1772). When external thread 1710 of lower shell 1706 and internal thread 1714 of lower shell fastener 1712 thread together, lower shell fastener 1712 locks the fastened component and lower shell 1706 together. Because a diameter of the flange forming external thread 1726 on upper shell (work body) 1724 is smaller than a smallest diameter of lower shell fastener 1712, the fastened component cannot escape the restraint of lower shell fastener 1712.

In use, a center of bottom connector 1704 or central opening 1705 is aligned with an acupoint first, and then strap 1702 is fastened to a desired body part such that the acupoint and bottom connector 1704 are in a concentric relationship. When we rotate upper cover 1750 (together with upper shell 1724), working end 1770 (having tip 1772) goes down. The work style of working end 1770 can be various, as described in Paragraph [0006], for example, it can cause the work extent sensor to sense a pressure, a temperature, or a depth etc. In the embodiment of Fig. 4, we assume that work extent sensor 1720 is a pressure sensor or a temperature sensor, circuit board 1730 senses its relevant parameters of pressure or temperature, and transmits an original signal, a converted signal or a computed signal. The so-called original signal, for example, is a pressure or a depth. The so-called converted signal or the computed signal, for example, can be a work intensity (such as an exposure intensity or a stimulus intensity) or a work performance (such as particle number released). It is better to explain here that to facilitate the user to align bottom connector 1704 or central opening 1705 with a specific body part or a specific acupoint, a total height of bottom connector 1704 is better controlled between 10% to 35% of a total height of the health-care device under a non-operating state.

In this embodiment, health-care device 1700 includes device body 1706 and work piece 1718 having operating end 1790 and opposite working end 1770, wherein working end 1770 is used to engage in a health-care work towards a specific part of a body part of a user, or an acupoint. Work body 1724 is connected to device body 1706 and mounts operating end 1790 on its lower part so that through operating operating end 1790 (or work body 1724), the user may maintain a specific working relationship between working end 1770 and the specific part or the acupoint. Device positioning piece 1702 (being flexible) connects thereto device body 1706 to be positioned on the body part such that a specific positional relationship between working end 1770 and the specific part or the acupoint is maintained. The so-called specific positional relationship, for example, is a pressing, a thermal stimulation, an electrical stimulation, or a light stimulation,... etc, such as those described in Paragraph [0006] of the present disclosure. Such specific positional relationship usually represents a work intensity of the health-care work, or an extent that working end 1770 or its tip 1772 approaches or touches the specific part or the acupoint.

To further understand Paragraph [0037] that the operating mode can be the various means as described in Paragraph [0006], we use the moxibustion as an example here to explain the practical use of the present disclosure. Upon investigation of the material of traditional Chinese moxibustion, moxa, its ingredients are found to include volatile oil, Flavonoids, Tannins, Polysaccharide and trace elements (iron, calcium and phosphorus), so that special effects are obtained. But, it needs a Chinese physician or a nurse to do it to prevent the patient from being scalded, and thus is inconvenient. Further, after infrared was discovered by the scientist William Herschel when he used the prism to study the thermal effect of spectrum in 1800, it is widely used in related medical care or rehabilitation. It can be further divided into near infrared, middle infrared and far infrared, and the wavelengths released by the infrared medical equipment are all in the range of the far infrared. The far infrared can have the effects on the organism of: generating a warm effect, promoting a blood circulation, improving a poor microvascular circulation, promoting a tissue growth and promoting a tissue regeneration. Thus, the far infrared not only can promote the growth and development of organisms, but also can be used as auxiliary treatment tools such as alleviating the pain of the wound, promoting the wound healing, activating a blood stasis, promoting the healing of diabetic foot ulcer, treating a high blood pressure and relieving mental stress,... etc. Recently, there is an academic research disclosing that the far infrared having the wavelength of 4-14µm is a fertility light, tourmaline is a natural ore releasing far Infrared and negative ions continuously, magnesioferrite crystal contained in the tourmaline is the key of releasing the far infrared, and there are materials synthesized by natural ores that release this far infrared. Even with this material, its inconvenience in use has no difference from the traditional acupuncture or moxibustion.

Further, the human body contains 70% water and 80% of blood is water. In addition, one of resonant wavelengths of the water is 6.27µm. Although the red infrared has a wide range (0.78-1000µm), only 4-14µm of the light of life cover the resonant wavelength of water, can trigger the resonance of water molecules, and crack big water molecular cluster into small molecule water for easy human body absorption. Still more, the human body is carbohydrate, the resonant wavelength of C-H chain, C-C chain and C-O chain are all in the range of the light of life, and thus the wavelength of 4-14µm can go deep into the capillaries via the human body molecular resonance so as to speed up the blood flow. After this introduction, no matter whether the moxibustion or the far infrared, both are desirable. However, to put them in use at low cost and with convenience, the present invention is indispensable, as described as follows.

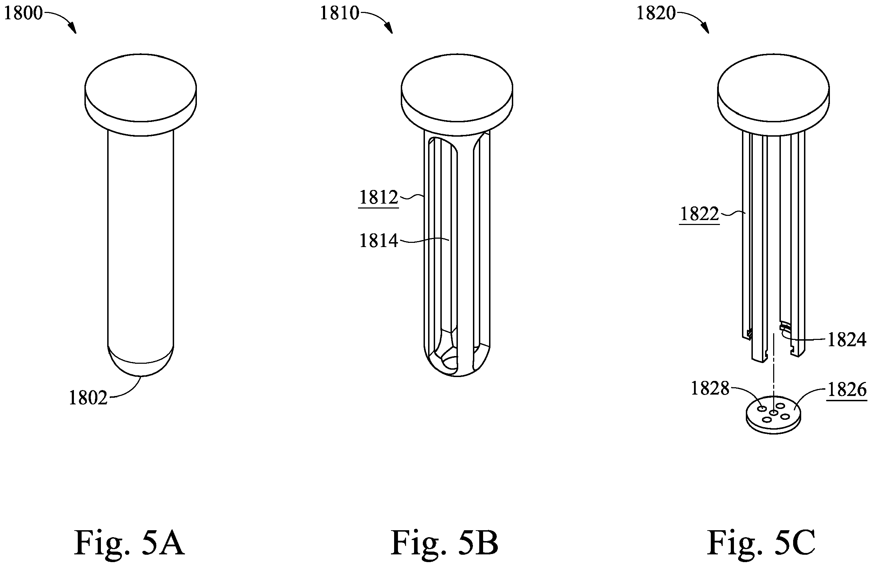

Please refer to Fig. 5A, which shows the first embodiment of work piece 1800 of the fourth embodiment of the health-care device having a work front 1802. Work piece 1800 is molded with the material of natural ore radiating far infrared as described in Paragraph [0038]. Replacing work piece 1718 in Fig. 4 with work piece 1800, obviously it can perform the far infrared care in a simple and yet low-cost way. Please refer to the embodiment in Fig. 4, through the extent that external thread part 1726 is screwed downward along internal thread 1708, we can assure a distance between work front 1802 and the acupoint or the skin surface to be health-cared, and adjust or set the relevant working parameters according to this distance.

Please refer to Fig. 5B, which shows the second embodiment of work piece 1810 of the fourth embodiment of the health-care device, wherein there are a plurality of longitudinal grooves 1814, through which, not only the far infrared releasing material can be saved, but the moxa can be simultaneously put into longitudinal grooves 1814 so as to obtain the dual effects. Please refer to Fig. 5C, which shows the third embodiment of work piece 1820 of the fourth embodiment of the health-care device, wherein work piece body 1822 includes four evenly and circularly arranged rectangular columns, each of which has a groove 1824 retaining thereby a far infrared releasing cake 1826 serving as the working end. There are a plurality of openings 1828 on cake 1826 capable of disposing the moxa thereon, by which the essence upon burning the moxa can be penetrated into the skin via openings 1828 as expected by a traditional moxibustion.

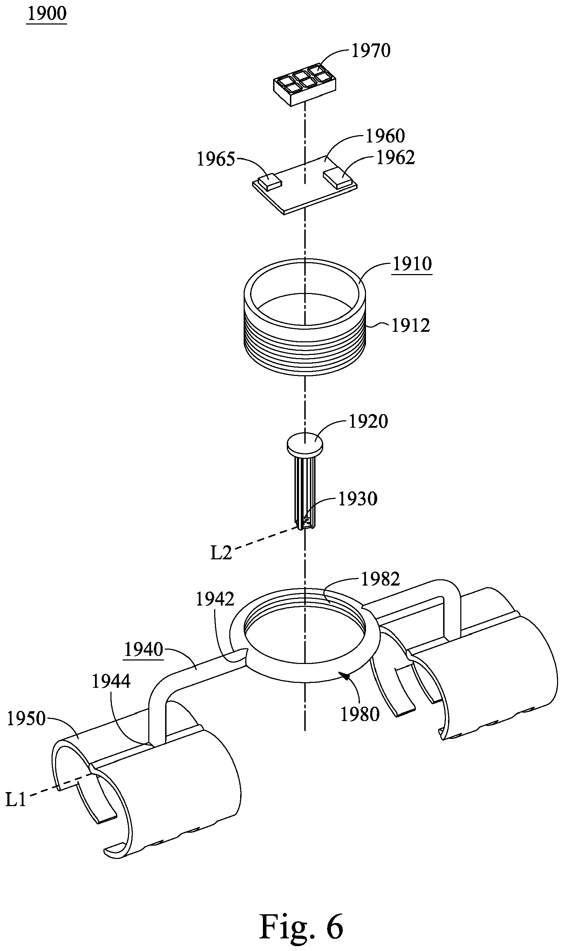

Usually, the human skin can stand a highest contact temperature of no harm is 45 degrees Celsius. At 53 degrees Celsius, it is very likely that you will get burned after one minute. When the burning moxa contacts the skin, it must cause a scald, and that is one of the reasons why needle is more popular than the moxibustion. Via circuit board 1730 and a precisely controlled working distance between work front 1802 and the skin of the body part, not only we can avoid contact burn, but also the working temperature of the cake can be increased. As is known, the higher the working temperature is, the more the far infrared is released by cake 1826, and thus the healing effect is increased. Certainly, under this embodiment, the structure must be different from the above-mentioned examples. As shown in Fig. 6, it is the fifth embodiment of the health-care device 1900 including a work body 1910 having an external thread 1912, a work piece 1920 retaining at its end a far infrared cake 1930, one or a pair of device positioning pieces 1940, a circuit board 1960, a heating device 1962, a heating controller 1965, a circular connector 1980 having an internal thread 1982, and a heating extent display 1970. Each device positioning piece 1940 has a first terminal 1942 connected to circular connector 1980, and connected to work body 1910 via screwing internal thread 1982 around external thread 1912, and a second terminal 1944 connected to an elastic limb retainer 1950. The level at which limb retainer 1950 contacts the limb skin is LI, and the level at the bottom of work piece is L2. We can control a distance between L1 and L2 easily, for example, to be 2mm or 1cm, and then define the working style of work piece 1920. Having gone through the explanations of so many embodiments, further explanations of more details should thus be redundant.

It is worth to mention that in the example of Fig. 6, because work piece 1920 is only required to be secured to work body 1910 (equivalent to work body or upper shell 1724 in Fig. 4), lower shell 1706 in Fig. 4 can be omitted. Interpreting otherwise, device body 1706 has been integrally formed with work body 1910 at this moment.

The present invention focuses on a method for operating a health-care device, but not the health-care device itself. Thus, the disclosure of the above-mentioned health-care device and its components only discusses few health-care devices suitable for using the present operating method. To explain in details, the core features of the health-care devices suitable for using the present operating method are as follows: a health-care device including a work piece having an operating end and an opposite working end, wherein the working end is used to engage in a health-care work towards a specific part of a body part of a user or an acupoint, wherein the working end has a tip, a work body mounts the operating end on its lower part to allow the user through operating the operating end or the work body to maintain a specific working relationship between the working end or the tip and the specific part or the acupoint, and a device positioning piece connected to the work body to allow the user to position the work body to the body part to maintain a specific positional relationship between the working end or the tip and the specific part or the acupoint.

The above-mentioned health-care device further includes a bottom fastener, and the means employed by the health-care work is one selected from a group consisting of an electrical stimulation, a light stimulation, a thermal stimulation, a non-invasive or a contactless acupoint stimulation mode, a microwave stimulation, a magnetic stimulation, an acupuncture needle, a moxibustion, and a combination thereof, wherein the bottom fastener is a connector connecting the work body to the device positioning piece, and/or the device positioning piece is a flexible element configured around the body part, or a mechanism element fixed to the body part.

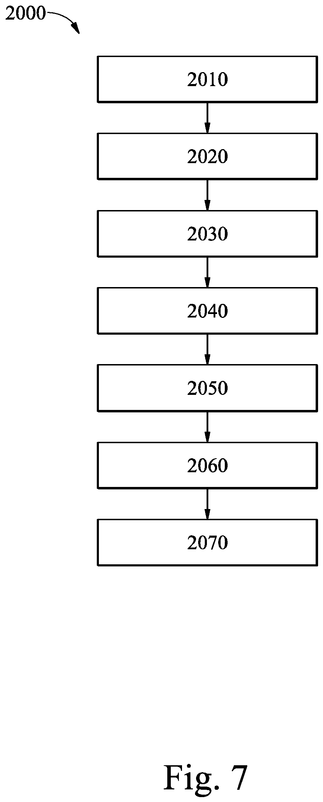

Some key features of the health-care devices for operation by the present invention may have been known. To facilitate the understanding of the present operating method, their embodiments are sequentially illustrated by referring to the health-care device in Fig. 4. Please refer to Fig. 7, which shows a method 2000 for operating a health-care device 1700 including device body 1706, work piece 1718, work body 1724, bottom fastener 1704 and device positioning piece 1702, wherein work piece 1718 has operating end 1790 and opposite working end 1770, working end 1770 has tip 1772, work body 1724 mounts operating end 1790 on its lower part 1725, bottom fastener 1704 has a central opening. Method 2000 includes a first step: 2010: attaching bottom fastener 1704 to device positioning piece 1702; a second step 2020: aligning central opening 1705 (of bottom fastener 1704 and device positioning piece 1702) with a specific part or an acupoint of a body part of a user; a third step 2030: fastening or fixing device positioning piece 1702 to the body part under the alignment between central opening 1705 and the specific part or the acupoint; a fourth step 2040: connecting device body 1706 to bottom fastener 1704; a fifth step 2050: securing work piece 1718 to work body 1724, and securing operating end 1790 to to lower part 1725 of work body 1724; a sixth step 2060: combining work body 1724 to device body 1706; and a seventh step 2070: operating operating end 1790 (secured to lower part 1725) to engage working end 1770 in a health-care work on the specific part or the acupoint, by which the user through operating the operating end 1790 (or work body 1724) to directly maintain working end 1770 or tip 1702 at a specific working relationship with the specific part or the acupoint, and to maintain working end 1770 or tip 1702 at a specific positional relationship with the specific part or the acupoint. Among which, first step 2010, fifth step 2050 and sixth step 2060 can be completed before leaving the factory, and the user only needs to operate the second to the fourth steps: 2020 to 2040 and the seventh step 2070.

The paragraphs above described the operating method using the Fig. 4 device as an example. If we use the Fig. 6 device as an example, its operating method will be even simpler because the device body and the work body are formed integrally. To explain this circumstance in details, the operating method will be as follows: because connector 1980 was combined to device positioning piece 1940, a first step is: adjusting a location of limb or body retainer 1950 on a body part to align the center of connector 1980 with a specific part or an acupoint of a body part of a user; a second step is: connecting work body 1910 to connector 1980; a third step is: securing work piece 1920 to work body 1910, and securing operating end 1930 to (a lower part of) work body 1910; and a fourth step is: operating work body 1910 to engage working end 1930 in a health-care work on the specific part or the acupoint, so that the user through operating the work body 1910 directly maintains working end 1930 at a specific working relationship with the specific part or the acupoint, and to maintain working end 1930 at a specific positional relationship with the specific part or the acupoint. As described in the paragraphs above, the third step can be completed before leaving the factory. In fact, the second step can also be initially completed before leaving the factory. Certainly, if the second step is directly omitted, and the initial completion is regarded as a partial step of the fourth step, it should be fully imaginable.

It was mentioned in Paragraph [0026] that the feeling of the light or heavy pressures borne by the acupoint might be quite different because of (1) age, (2) physique and (3) a coarseness or a tenderness of skin or muscle texture. In fact, the Applicant has conducted a research regarding this issue, and found that there are some differences as shown in the following Table 1 (where the so-called light pressure indicates that an acupoint was aware of a light touch or a light massage, and the so-called heavy pressure indicates that an acupoint was aware of a heavy touch or a heavy massage):

**Table 1**

| Acupoint | Body part | Average experimental values of children age 7 to 10 | | Average experimental values of adults | |
|---|---|---|---|---|---|
| | | Values of light pressure | Values of heavy pressure | Values of light pressure | Values of heavy pressure |
| He Gu | hand | 2.6487 | 3.8259 | 2.3544 | 3.8259 |
| Neiguan | | 1.7658 | 3.2373 | 1.4715 | 2.6487 |
| Waiguan | | 1.4715 | 2.0601 | 2.0601 | 2.6487 |
| Quze | | 1.7658 | 2.943 | 2.0601 | 3.2373 |
| Shaofu | | 2.3544 | 2.0601 | 1.7658 | 2.6487 |
| Shenmen | | 1.1772 | 2.0601 | 1.4715 | 2.6487 |
| Yanglao | | 1.1772 | 2.3544 | 2.0601 | 2.6487 |
| Zhongchong | | 1.1772 | 1.4715 | 0.5886 | 1.1772 |
| Jianjing | shoulder | 2.0601 | 3.2373 | 2.943 | 3.5316 |
| Shenfeng | chest | 1.4715 | 1.7658 | 1.7658 | 2.3544 |
| Guanyuan | abdomen | 1.7658 | 3.2373 | 1.7658 | 2.6487 |
| Shangqu | | 1.7658 | 2.0601 | 1.4715 | 2.0601 |
| Zhiyang | back | 0.8829 | 2.6487 | 1.4715 | 2.0601 |
| Zusanli | foot | 2.3544 | 3.5316 | 2.6487 | 3.2373 |
| Weizhong | | 1.1772 | 2.0601 | 2.3544 | 2.943 |
| Kunlun | | 1.7658 | 3.2373 | 1.7658 | 2.6487 |
| Dazhong | | 1.4715 | 2.6487 | 1.7658 | 2.6487 |
| Shuiquan | | 1.7658 | 3.2373 | 1.7658 | 2.6487 |
| Chengshan | | 1.4715 | 2.943 | 2.943 | 3.5316 |
| Pucan | | 2.3544 | 2.943 | 1.4715 | 2.3544 |

| | | | | | |
|---|---|---|---|---|---|
| Note: The unit of depth is mm, and the unit of pressure is N. | | | | | |

For caution's sake, the Applicant further conducts a study regarding whether the female is different from the male, and the obtained results are listed in Table 2.

**Table 2**

| Measured average pressure values of adult female | | | | | |
|---|---|---|---|---|---|
| Acupoint | Body part | Values of light pressure | Values of heavy pressure | Deviation of light pressure | Deviation of heavy pressure |
| Fengchi | head | 0.8829 | 1.1772 | 0.6231 | 0.2943 |
| Qiangjian | | 1.1772 | 1.4715 | 0 | 0 |
| He Gu | hand | 2.3544 | 3.5316 | 0 | -0.2943 |
| Neiguan | | 1.7658 | 2.3544 | 0 | -0.2943 |
| Chize | | 1.7658 | 2.943 | 0 | 0.2943 |
| Laogong | | 2.0601 | 2.943 | -0.5886 | -0.5886 |
| Sanyinjiao | foot | 1.7658 | 2.6487 | 0 | -0.2943 |
| Weizhong | | 2.0601 | 2.6487 | -0.2943 | -0.2943 |
| Yinlingquan | | 1.4715 | 2.0601 | 0 | 0 |
| Zusanli | | 1.7658 | 2.6487 | -0.8829 | -0.5886 |
| Average deviation | | | | -0.11427 | -0.17658 |
| | | | | | |

| Measured average pressure values of adult male | | | | | |
|---|---|---|---|---|---|
| Acupoint | Body part | Values of light pressure | Values of heavy pressure | | |
| Fengchi | head | 0.2598 | 0.8829 | | |
| Qiangjian | | 1.1772 | 1.4715 | | |
| He Gu | hand | 2.3544 | 3.8259 | | |
| Neiguan | | 1.7658 | 2.6487 | | |
| Chize | | 1.7658 | 2.6487 | | |
| Laogong | | 2.6487 | 3.5316 | | |
| Sanyinjiao | foot | 1.7658 | 2.943 | | |
| Weizhong | | 2.3544 | 2.943 | | |
| Yinlingquan | | 1.4715 | 2.0601 | | |
| Zusanli | | 2.6487 | 3.2373 | | |

| | | | | | |
|---|---|---|---|---|---|
| Note: The unit of depth is mm, and the unit of pressure is N. | | | | | |

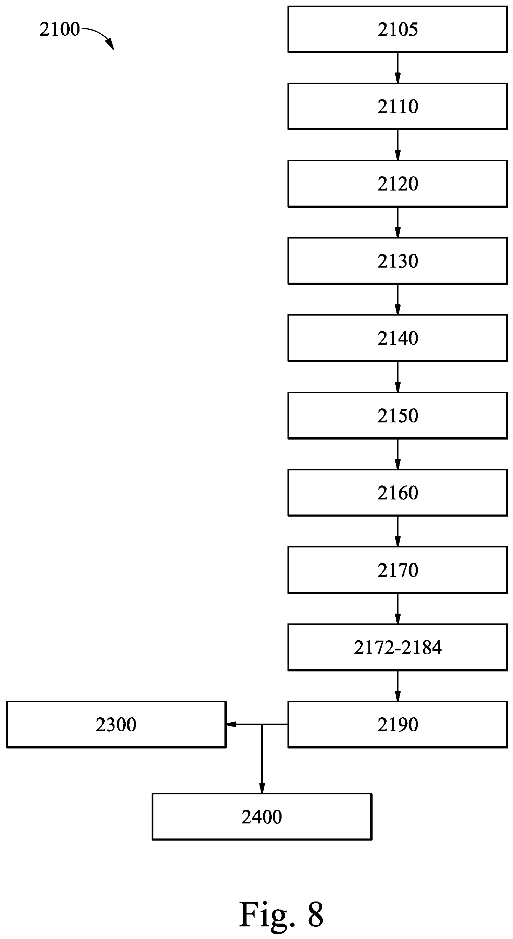

After engaging in-depth research and experiments, and explaining with the above examples, the Applicant considered that those age 6 to age 18 should be prorated to decrease at most 20% (of average values of the adult male) as their personal default values, the female should decrease at most 10% of the average values of the adult male as their personal default values, and the intersection of the two (female age less than 18) should decrease at most 20% of those as their personal default values. The purpose of this consideration is that when we design an APP, the user can use his/her mobile phone to operate health-care device 1700 to obtain the most suitable health-care work or pressing work. Fig. 8 is the second embodiment of the present operating method 2100. Under the APP designed by the Applicant, the user operates a first step 2110: aligning the central opening with a specific part or an acupoint of a body part of a user; a second step 2120: fastening or fixing the device positioning piece to the body part for maintaining an alignment between the central opening and the specific part or the acupoint; a third step 2130: connecting or fastening the device body to the bottom fastener; and a fourth step 2140: requesting the user to input his/her age and sex to adjust the default working parameters firstly.

The parameter adjustment only relates to age and sex, while physique and coarseness or a tenderness mentioned in Paragraph [0026] are not yet considered. For this reason, before or after fourth step 2140, we add an enquiry of a fifth step 2150: requesting the user to self-evaluate a body weakness/strength index for an adjustment of a predetermined working parameter. If the self-evaluation is strong, use the default value. On the contrary, if the self-evaluation is weak or has a tender skin or muscle texture, 10% from the default value is decreased as the secondly adjusted personal default parameter.

After the above-mentioned adjustments, we still wonder whether the secondly adjusted default parameters are the best temporary or long-term default parameters. Thus, we had better design that each time when the user uses health-care device 1700, the user is allowed to try again or successively to decide or find out whether the secondly adjusted default parameters should be further increased or decreased. For this reason, before or after fourth step 2140 and fifth step 2150, a sixth step 2160 is added: requesting the user, when compared to the preceding work or use, whether the user hopes that an intensity of the current work is to be further increased or decreased by 10%. For safety reasons, the user is allowed to increase the intensity by 20% at most, that is to set the limits of the default parameters (i.e. the upper limit for safety purpose, while the lower limit for ensuring a minimum effect to be happened). Certainly, here, the so-called before or after a specific step is not an absolute concept. For example, even we set the above-mentioned sixth step as the first step of using the health-care device, it is also not prohibited.

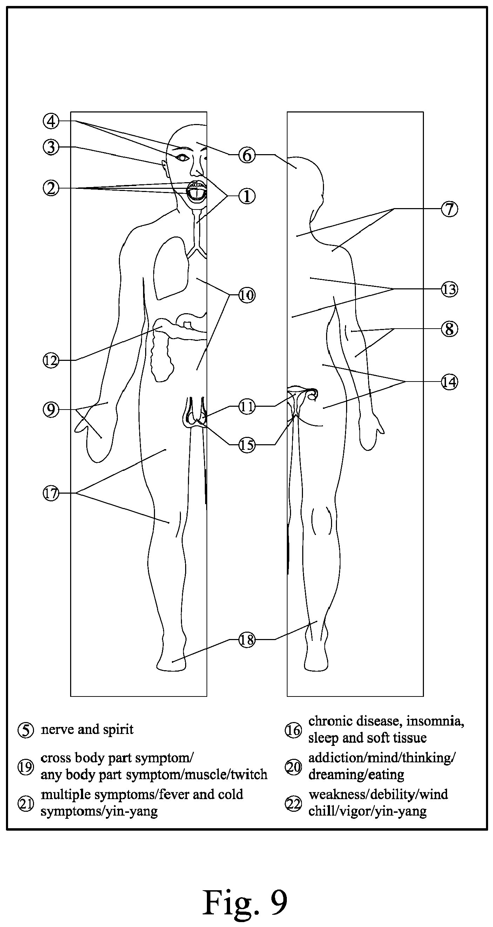

To further increase the convenience for the user, please refer to Fig. 9, which is an embodiment of a mobile phone screen showing human body with body parts where one may feel unwell or have diseases and symptoms for selection. In Fig. 9, the unwell body parts and symptoms are categorized into 22 types including physical body parts and spiritual classifications. That is (1) nose and respiratory system, (2) mouth, tooth and throat-related, (3) ear-related, (4) eye and eyebrow-related, (5) nerve and spirit, (6) head and face, (7) shoulder and neck, (8) elbows and joints, (9) palm and wrist, (10) chest and abdomen, (11) delivery and reproductive systems, (12) digest system, (13) back/spine/tendons/bones, (14) waist/buttocks (lower back), (15) genitals and anus, (16) chronic disease, insomnia, sleep and soft tissue, (17) legs, knees and joints, (18) sole and ankle, (19) plural body parts/muscle/twitch, (20) addiction/mind/thinking/dreaming/eating, (21) multiple symptoms/fever and shiver/yin-yang, and (22) weakness/debility/evil wind/qi/yin-yang. After the user's initial selection of one of the 22 types, the APP then gets from the database all possible diseases for the selected type. When the user selects a specific disease again from the all possible diseases in a specific type, the APP further gets from the database all possible acupoints that can be used to treat the specific disease for choice by the user again. The database was built in the websites of the Applicant's related companies. They are the public websites of TaoBody Lifetime Healthiness, Inc. (www.tao-body.com) and WeiDui, Inc. (www.taobody.com.tw). What is listed in the below Table 3 is a portion of its database having the format as follows:

**Table 3**

| Acupoint | Meridian | Body part | Disease |
|---|---|---|---|
| Jingqu | (Hand greater yin Lung Meridian) LU | 2 | Asthma, vomiting, yawning, stretching |
| Jingqu | (Hand greater yin Lung Meridian) LU | 13 | Tight back |
| Binao | Hand yangming Large Intestine Meridian) LI | 7 | Spastic torticollis/stiff and tight neck |
| Binao | (Hand yangming Large Intestine Meridian) LI | 4 | Epidemic hemorrhagic conjunctivitis (sudden tears and redness of the eye white/acute conjunctivitis) |
| Binao | (Hand yangming Large Intestine Meridian) LI | 4 | Eye diseases externally caused (acute swelling and pain in eye) |
| Binao | (Hand yangming Large Intestine Meridian) LI | 4 | Punctate keratitis (virus keratitis of simplex herpes) |
| Zhonglushu | (Foot greater yang Bladder Meridian) BL | 2 | Diabetes |
| Zhonglushu | (Foot greater yang Bladder Meridian) BL | 21 | Diabetes |
| Zhonglushu | (Foot greater yang Bladder Meridian) BL | 15 | Red and white diarrhea |
| Jinmen | (Foot greater yang Bladder Meridian) BL | 21 | Suddenly fainted (weak breathing, and very thin pulse) |
| Jinmen | (Foot greater yang Bladder Meridian) BL | 21 | Shaking head and opening mouth |
| Fengshi | (Foot lesser yang Gallbladder Meridian) GB | 3 | Deaf/tinnitus |
| Fengshi | (Foot lesser yang Gallbladder Meridian) GB | 17 | Short/tight hamstrings |
| Baihuanshu | (Foot greater yang Bladder Meridian) BL | 14 | Lower back keeping pain/cold pain in lumbar spine |
| Chengfu | (Foot greater yang Bladder Meridian) BL | 14 | Cold and severe pains in lumbar, spine, thigh and buttocks |
| Chengfu | (Foot greater yang Bladder Meridian) BL | 11 | Cold in the uterus |
| Zhongzhu | (Foot lesser yin Kidney Meridian) KI | 15 | Hard and dry stool uneasy to be discharged |
| Shangqu | (Foot lesser yin Kidney Meridian) KI | 10 | Often acute pain due to blockade in abdomen/acute pain in intestines |
| Xiyangguan | (Foot lesser yang Gallbladder Meridian) GB | 17 | Pain in outer side of the knee |
| Xiyangguan | (Foot lesser yang Gallbladder Meridian) GB | 19 | Lymphatic tuberculosis |
| Heyang | (Foot greater yang Bladder Meridian) BL | 17 | Gastrocnemius spasm/calf cramps |
| Heyang | (Foot greater yang Bladder Meridian) BL | 11 | Dysmenorrhea (abdominal pain during menstruation) |
| Heyang | (Foot greater yang Bladder Meridian) BL | 18 | Cold heel and tight knee |

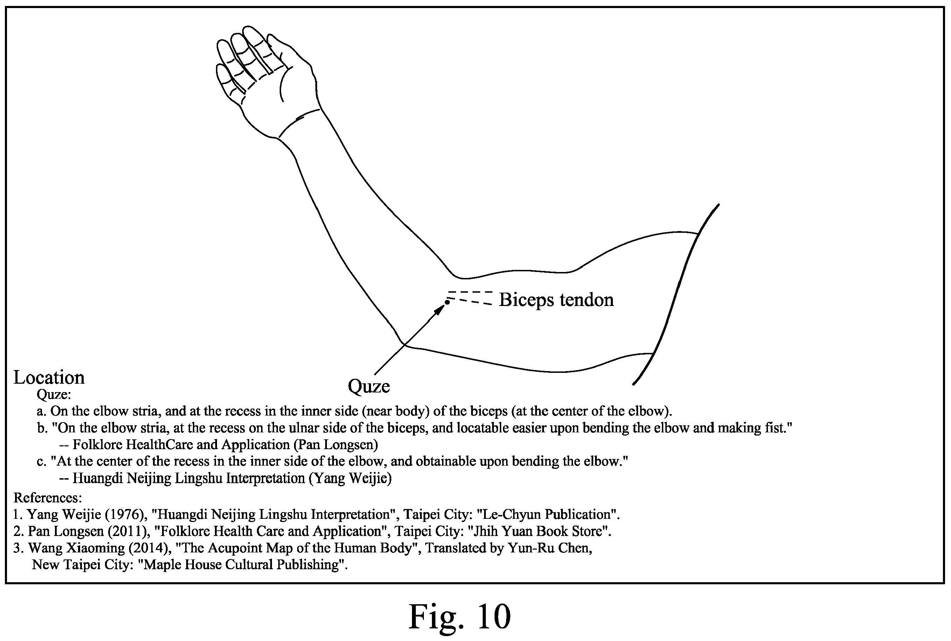

To the above end, before or after fourth step 2140, fifth step 2150 or sixth step 2160, a seventh step 2170 is added: requesting the user to select sequentially a body part feeling unwell or having a disease or a symptom, a specific illness or symptom that he believes his body part is suffering, and a working acupoint he plans to use. According to the selections he made, as exemplified in Fig. 10, the figure (a real picture when the APP is used) and descriptions of the Quze acupoint of The Hand-Jueyin Pericardium Meridian are available. That is, the APP will get the picture of Quze shown by a real man from the above websites so that from the mobile phone, the user can clearly know and locate where the selected acupoint is, through additional assistance from textual descriptions quoted from inter alia the classical medical books.

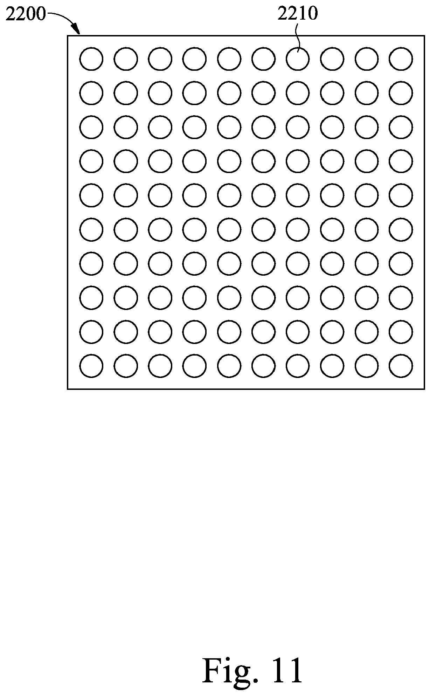

Fig. 11 shows a sticker sheet 2200 with plural circular stickers 2210, each of which can be used to pre-position a location of an acupoint. Before or after first step 2110 and second step 2120, there is a preparation step 2105: Attaching a circular sticker 2210 on a specific part or acupoint by the user after consulting the acupoint picture and descriptions shown on the mobile phone screen. Thus, either the alignment of first step 2110 or pre-positioning the specific part or the acupoint of second step 2120 will become much easier.

After the preparations above, the user can engage in the core operation step 2190: operating the operating end 1790 (work body 1724) to engage working end 1770 or tip 1772 in a health-care work on the specific part or the acupoint, so that through operating the operating end 1790 (or work body 1724), the user may directly maintain a specific working relationship between working end 1770 or tip 1772 and the specific part or the acupoint, and maintain a specific positional relationship between working end 1770 or tip 1772 and the specific part or the acupoint. The specific working relationship indicates the content or extent of the health-care, e.g., pressing, illumination, or moxibustion etc, as described in Paragraph [0006]. The specific positional relationship indicates the geometric relationship, or spacing, contacting, or positional relationships between working end 1770 or tip 1772 with the specific part or the acupoint (skin surface).

The disclosure regarding the present method for operating a health-care device should have been complete, but steps of common sense or routine life can also be introduced. For example, a step 2172 of setting a working strength; a step 2174 of setting a working or administering time; a step 2176 of setting a use frequency being an interval of two uses; a step 2178 of reminding this use time is up; a step 2180 of setting a mobile phone to remind or to cease to remind of a next use time; a step 2182 of causing a mobile phone to display an extent of the working strength having reached a certain percentage,... etc. Certainly, to be more refined, we can have a step 2184 of asking the user to identify an ill or discomforting situation which is currently (A) in an initial period, (B) in a serious period, (C) in a recovering period, or (D) a chronic disease, for corresponding adjusting the working or administering time and interval. Because such introduction or refinement is an imaginable workmanship of the Chinese physician, it is hardly exhaustible here.

In sum, the present method mainly includes: aligning a positioning opening of a bottom connector or a device connector with a specific part or an acupoint of a body part of a user; attaching or fixing a device positioning piece to the body part for maintaining an alignment between the positioning opening and the specific part or the acupoint; connecting a work body to the device positioning piece; and operating an operating end or the work body to engage a working end in a health-care work on the specific part or the acupoint, so that through operating the operating end or the work body, the user directly maintains a specific working relationship between the working end or the tip and the specific part or the acupoint, and maintains a specific positional relationship between the working end or the tip and the specific part or the acupoint. Certainly, the above-mentioned four steps can be performed sequentially; and/or the user can directly operate the operating end or the work body; and/or the working end or the tip can directly maintain a specific working relationship with the specific part or the acupoint.

While the invention has been described in terms of what is presently considered to be the most practical and preferred embodiments, it is to be understood that the invention need not be limited to the disclosed embodiments. Therefore, it is intended to cover various modifications and similar configurations included within the spirit and scope of the appended claims, which are to be accorded with the broadest interpretation so as to encompass all such modifications and similar structures.

## Claims

1. A method for operating a health-care device (1), wherein the health-care device (1) includes a device body (1706), a work piece (20, 380, 1718, 1730, 1800, 1810, 1820, 1920), a work body (10, 390, 1724, 1910) having an upper part (1727) and a lower part (1725), a bottom connector (1704) and a device positioning piece (1702, 1940), the work piece (20, 380, 1718, 1730, 1800, 1810, 1820, 1920) has an operating end (1790, 1930) and an opposite working end (24, 384, 1770, 1930), the working end (24, 384, 1770, 1930) has a tip (24T, 702, 1772), the work body (10, 390, 1724, 1910) mounts the operating end (1790, 1930) on the lower part (1725) and is connected to the device body (1706), and the bottom connector (1704) has a central opening (1705), **characterized by** comprising:
coaxially aligning the central opening (1705) to a specific part or an acupoint of a body part of a user;
causing the device positioning piece (1702, 1940) to be attached to the body part for maintaining an alignment between the central opening (1705) and the specific part or the acupoint;
causing the device body (1706) to be attached to the bottom connector (1704); and
operating the operating end (1790, 1930) or the work body (10, 390, 1724, 1910) to engage the working end (24, 384, 1770, 1930) in a health-care work on the specific part or the acupoint, to maintain by the user through operating the operating end (1790, 1930) or the work body (10, 390, 1724, 1910) a specific working relationship between the working end (24, 384, 1770, 1930) or the tip (24T, 702, 1772) and the specific part or the acupoint, and to maintain a specific positional relationship between the working end (24, 384, 1770, 1930) or the tip (24T, 702, 1772) and the specific part or the acupoint.

2. The method for operating the health-care device (1) according to Claim 1, **characterized in that** the bottom connector (1704) is a bottom fastener (1704), and the method further comprises the following step:
causing the bottom fastener (1704) to be combined to the device positioning piece (1702, 1940).

3. The method for operating the health-care device (1) according to Claim 1 or 2, **characterized in that** the device body (1706) and the work body (10, 390, 1724, 1910) are formed integrally, and the method further comprises the following step:
requesting the user to input an age and/or a sex thereof for an adjustment of a predetermined working parameter therefor.

4. The method for operating the health-care device (1) according to any of Claims 1 - 3, **characterized in that** the specific working relationship indicates an extent that the working end (24, 384, 1770, 1930) or the tip (24T, 702, 1772) approaches or touches the specific part or the acupoint.

5. The method for operating the health-care device (1) according to any of Claims 1 - 4, **characterized by** further comprising the following step:
requesting the user to self-evaluate a body weakness/strength index for an adjustment of a predetermined working parameter.

6. The method for operating the health-care device (1) according to any of Claims 1 - 5, **characterized in that** the working end (24, 384, 1770, 1930) or the tip (24T, 702, 1772) directly maintains the specific working relationship with the specific part or the acupoint.

7. The method for operating the health-care device (1) according to any of Claims 1 - 6, **characterized by** further comprising the following steps:
requesting the user to set a desired working strength of a current health-care work, by increasing, maintaining or decreasing a specific percentage of a working strength used in a previous health-care work; and/or
setting limits for health-care working parameters.

8. The method for operating the health-care device (1) according to any of Claims 1 - 7, **characterized in that** a total height of the bottom connector (1704) is between 10% to 35% of a total height of the health-care device (1) under a non-operating state.

9. The method for operating the health-care device (1) according to any of Claims 1 - 8, **characterized by** further comprising the following steps:
requesting the user to select sequentially an ill or discomforting body part from a body image, a concrete illness or symptom on the ill or discomforting body part, and a desired working acupoint; and/or
downloading a picture and textual descriptions of the specific part or the acupoint from a database to a mobile device of the user.

10. The method for operating the health-care device (1) according to any of Claims 1 - 9, **characterized in that** the specific positional relationship indicates one of a distance, a contacting relationship and an orientation between the working end (24, 384, 1770, 1930) or the tip (24T, 702, 1772) and the specific part or the acupoint.

11. The method for operating the health-care device (1) according to any of Claims 1 - 10, **characterized by** further comprising a preparation step of sticking a round sticker to the specific part or the acupoint.

12. The method for operating the health-care device (1) according to Claim 1, **characterized by** further comprising a step being one selected from a group consisting of:
a step of setting a working strength;
a step of setting a working or administering time;
a step of setting a use frequency being an interval of two uses;
a step of setting a mobile phone to remind or to cease to remind of a next use time;
a step of causing a mobile phone to display an extent of the working strength has reached a certain percentage; and
allowing the user to identify an ill or discomforting situation is currently (A) in an initial period, (B) in a serious period, (C) in a recovering period, or (D) a chronic disease, so as to accordingly adjust the working or administering time and the interval.

13. A method for operating a health-care device (1), **characterized in that** the health-care device (1) includes a work piece (20, 380, 1718, 1730, 1800, 1810, 1820, 1920), a work body (10, 390, 1724, 1910) and a device positioning piece (1702, 1940), the work piece (20, 380, 1718, 1730, 1800, 1810, 1820, 1920) has an operating end (1790, 1930) and an opposite working end (24, 384, 1770, 1930), the working end (24, 384, 1770, 1930) has a tip (24T, 702, 1772), the device positioning piece (1702, 1940) has an aligning opening, and the method comprises:
coaxially aligning the aligning opening with a specific part or an acupoint of a body part of a user;
fixing the device positioning piece (1702, 1940) to the body part for maintaining an alignment relationship between the aligning opening and the specific part or the acupoint;
attaching the work body (10, 390, 1724, 1910) to the device positioning piece (1702, 1940); and
operating the operating end (1790, 1930) or the work body (10, 390, 1724, 1910) to engage the working end (24, 384, 1770, 1930) or the tip (24T, 702, 1772) in a health-care work on the specific part or the acupoint, to maintain by the user through operating the operating end (1790, 1930) or the work body (10, 390, 1724, 1910) a specific working relationship between the working end (24, 384, 1770, 1930) or the tip (24T, 702, 1772) and the specific part or the acupoint, and to maintain a specific positional relationship between the working end (24, 384, 1770, 1930) or the tip (24T, 702, 1772) and the specific part or the acupoint.

14. The method for operating the health-care device (1) according to Claim 13, wherein the four steps are operated sequentially, the work body (10, 390, 1724, 1910) has an upper part (1727) and a lower part (1725), the operating end (1790, 1930) is mounted on the lower part (1725), the user directly operates the operating end (1790, 1930) or the work body (10, 390, 1724, 1910), the working end (24, 384, 1770, 1930) or the tip (24T, 702, 1772) directly maintains the specific working relationship with the specific part or the acupoint, the health-care device (1) further comprises a bottom connector (1704) or retainer (1950), and the health-care work or the specific working relationship is one selected from a group consisting of an electrical stimulation, a light stimulation, a thermal stimulation, a non-invasive or a contactless acupoint stimulation mode, a microwave stimulation, a magnetic stimulation, an acupuncture needle, a moxibustion, and a combination thereof, wherein:
the bottom connector (1704) or retainer (1950) has a central opening (1705), the central opening (1705) and the aligning opening are coaxial or coincide with each other, a total height of the bottom connector (1704) or retainer (1950) is between 10% to 35% of a total height of the health-care device (1) under a non-operating state, and the bottom connector (1704) or retainer (1950) connects the work body (10, 390, 1724, 1910) to the device positioning piece (1702, 1940).

15. The method for operating the health-care device according to any of Claim 13 or 14, **characterized in that** the device positioning piece (1702, 1940) is flexible and mounted around the body part, or a member fixed on the body part.
